# EUROPEAN PATENT SPECIFICATION

(11) **EP 2 766 330 B1**
(45) Date of publication and mention of the grant of the patent: **18.03.2020**
(21) Application number: 12840366.4
(22) Date of filing: 10.10.2012
(51) Int. Cl.: C07C 17/20, C07C 17/25, C07C 21/18, C07C 17/087

(54) **PROCESS FOR PRODUCING 2,3,3,3-TETRAFLUOROPROPENE**
VERFAHREN ZUR HERSTELLUNG VON 2,3,3,3-TETRAFLUORPROPEN
PROCÉDÉ POUR LA PRÉPARATION DE 2,3,3,3-TÉTRAFLUOROPROPÈNE

(30) Priority: 14.10.2011 US 201161547219 P
(43) Date of publication of application: 20.08.2014
(73) Proprietor: Honeywell International Inc., Morris Plains, NJ 07950 (US)
(72) Inventor: WANG, Haiyou, Amherst, NY 14228 (US); TUNG, Hsueh Sung, Getzville, NY 14068 (US); MERKEL, Daniel C., West Seneca, NY 14224 (US)
(74) Representative: Stepney, Gregory John
(86) International application number: PCT/US2012/059437
(87) International publication number: WO 2013/055722

(56) References cited:
- EP-A1- 2 149 543
- WO-A1-2011/087825
- US-A1- 2009 240 090
- US-A1- 2010 029 997
- US-A1- 2010 036 179
- US-A1- 2010 331 583
- US-B2- 8 252 964

## Description

### FIELD OF INVENTION

This invention relates to a process for producing 2,3,3,3-tetrafluoropropene (HFO-1234yf).

### BACKGROUND OF THE INVENTION

Hydrofluoroolefins (HFOs), such as tetrafluoropropenes (including 2,3,3,3-tetrafluoropropene (HFO-1234yf)), are now known to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFOs do not contain chlorine and, thus, pose no threat to the ozone layer. HFO-1234yf has also been shown to be a low global warming compound with low toxicity and, hence, can meet increasingly stringent requirements for refrigerants in mobile air conditioning. Accordingly, compositions containing HFO-1234yf are among the materials being developed for use in many of the aforementioned applications.

Several methods of preparing HFOs are known. For example, U.S. Patent No. 4,900,874 (Ihara et al.) describes a method of making fluorine containing olefins by contacting hydrogen gas with fluorinated alcohols. Although this appears to be a relatively high-yield process, commercial scale handling of hydrogen gas at high temperature is hazardous. Also, the cost of commercially producing hydrogen gas, such as building an on-site hydrogen plant, is economically costly.

U.S. Patent No. 2,931,840 (Marquis) describes a method of making fluorine containing olefins by pyrolysis of methyl chloride and tetrafluoroethylene or chlorodifluoromethane. This process is a relatively low yield process and a very large percentage of the organic starting material is converted to unwanted and/or unimportant byproducts, including a sizeable amount of carbon black which tends to deactivate the catalyst used in the process. US-2009/240090 and US-2010/331583 also relate to the production of HFO-1234yf.

The preparation of HFO-1234yf from trifluoroacetylacetone and sulfur tetrafluoride has been described (See Banks, et al., Journal of Fluorine Chemistry, Vol. 82, Iss. 2, p. 171-174 (1997)). Also, U.S. Patent No. 5,162,594 (Krespan) discloses a process wherein tetrafluoroethylene is reacted with another fluorinated ethylene in the liquid phase to produce a polyfluoroolefin product.

However, there remains a need for an economic means of producing hydrofluoroolefins, such as HFO-1234yf. The present invention satisfies this need among others.

### SUMMARY OF THE INVENTION

The present invention relates, in part, to the surprising discovery that, during the dehydrochlorination of 2-chloro-1,1,1,2-tetrafluoropropane (HCFC-244bb) to HFO-1234yf in a reactor, impurities formed through halidation of the materials of the reactor leads to a reduction of the HFO-1234yf production rate and/or selectivity changeover from HFO-1234yf to 2-chloro-3,3,3-trifluoropropene (HCFO-1233xf). Accordingly, the present invention relates to methods of improving HFO-1234yf production and selectivity by reducing the presence of impurities in the reactor and avoiding, or at least reducing, the formation of HCFO-1233xf.

In one aspect, the present invention relates to a process for preparing 2,3,3,3-tetrafluoropropene comprising:
(a) providing a reaction to make 2,3,3,3-tetrafluoropropene by dehydrochlorination of 2-chloro-1,1,1,2-tetrafluoropropane, the reaction taking place in a reactor, the reaction having a first selectivity for 2,3,3,3-tetrafluoropropene;
(b) detecting a second selectivity for 2,3,3,3-tetrafluoropropene as the reaction proceeds;
(c) ceasing the reaction;
(d) removing impurities from said reactor by introducing a reducing agent or an oxidizing agent or by mechanical means under conditions effective to reduce the impurities and improve selectivity to 2,3,3,3-tetrafluoropropene; and
(e) resuming the reaction.

As used herein, the definition of "substantially free" means that the amount of impurities is reduced in the reactor so as to measurably improve the HFO-1234yf production rate and/or selectivity of the conversion of HCFC-244bb to HFO-1234yf. It may also mean the decrease in the conversion of HCFC-244bb to HCFO-1233xf. The impurities may include, but are not limited to, metal halides, metal oxides, and/or carbonaceous materials. The metal halides can comprise, for example, halides of Ni, Cr, Fe, Mo, Nb, Cu, and Co.

In certain embodiments, the step of removing impurities from the reactor comprises introducing a reducing agent into the reactor under conditions effective to convert any metal halides or metal oxides into metallic metals. Such reducing agents may include, but are not limited to, H₂, NH₃, CO, C₁-C₁₂ hydrocarbons, and combinations of these. The reducing agent can be in pure or in diluted form, e.g. diluted with inert gases. The dilution can be as high as practically possible, for example about 0.1% volume of reducing agent. This step may be performed alone or in conjunction with any other step for removing impurities. In certain embodiments, reducing occurs under conditions of continuous flow of reducing agent. In one embodiment, the reactor thus subjected to reducing can be afterward kept under positive pressure, or be sealed, with reducing agent and/or inert gas.

In alternative embodiments, the step of removing impurities from the reactor comprises introducing an oxidizing agent into the reactor under conditions effective to burn off the carbonaceous materials in the reactor. The oxidizing agent, for example, can comprise, but are not limited to, H₂O, CO₂, O₂ (oxygen), air, O₃, Cl₂, N₂O, and combinations of these. This step may be performed alone or in conjunction with any other step for removing impurities. In one embodiment, oxidizing occurs under conditions of continuous flow of oxidizing agent. In another embodiment, the oxidizing agent is diluted or provided in diluted form. Suitable diluents include inert gases, including, for example, He, Ar, N₂, and combinations of these. In one practice of this embodiment, the oxidizing agent is oxygen present in air and is diluted with nitrogen. The dilution can be as high as practically possible, for example about 0.1% volume of oxidizing agent.

In certain embodiments, introduction of an oxidizing agent is performed immediately preceding the introduction of the reducing agent; in certain embodiments of this practice, there is an interceding purge step between the oxidation and reduction. In certain embodiments, the purge step involves introducing inert gas into the reactor, inert gases including, for example, He, Ar, N₂, and combinations of these. In certain embodiments, the reactor is purged with the inert gas at a temperature of from about 100°C to about 700°C, about 200°C to about 600°C, about 300°C to about 500°C, or about 400°C to about 500°C. In one embodiment, purging is performed under conditions of continuous flow of inert gas. In another embodiment, the reactor is pressurized with inert gas and then depressurized for a few times sufficient to accomplish purging. Combinations of these embodiments may also be implemented.

In further alternative embodiments, the step of removing impurities from the reactor comprises physically removing carbonaceous materials and metal halides from the reactor. The physical removal can be achieved by, for example, electrical polishing and/or mechanical polishing of the interior surface of the reactor. In another example, these impurity deposits can be washed away with high flow rate of water or steam. This step may be performed alone or in conjunction with any other step for removing impurities.

In certain embodiments, a starting composition comprising HCFC-244bb is contacted with a dehydrochlorination catalyst occurs in the vapor phase. The catalyst, for example, may be selected from the group consisting of (i) one or more halides, (ii) one or more halogenated metal oxides, (iii) one or more zero-valent metals/metal alloys, and (iv) a combination of two or more of these. In certain embodiments, the selectivity to HFO-1234yf is at least 90% or higher, 95% or higher, or 97% or higher.

In even further aspects, the present invention relates to a process for preparing HFO-1234yf by
(i) providing a starting composition including a compound of Formulas I, II, or III:

   CX₂=CCl-CH₂X (I);

   CX₃-CCl=CH₂ (II);

   or

   CX₃-CHCl-CH₂X (III)

   wherein X is independently selected from F, Cl, Br, and I, provided that at least one X is not fluorine;
(ii) contacting the starting composition with a first fluorinating agent to produce a first intermediate composition including 2-chloro-3,3,3-trifluoropropene;
(iii) contacting the first intermediate composition with a second fluorinating agent to produce a second intermediate composition including 2-chloro-1,1,1,2-tetrafluoropropane; and
(iv) dehydrochlorinating at least a portion of the 2-chloro-1,1,1,2-tetrafluoropropane to produce a reaction product including 2,3,3,3-tetrafluoropropene, wherein the dehydrochlorination step is performed in accordance with the subject-matter described in the claims.

Additional embodiments and advantages to the present invention will be readily apparent to one of skill in the art, based on the disclosure provided herein.

### BRIEF DESCRIPTION OF THE FIGURES

FIG. 1 illustrates conversion of HCFC-244bb conversion and product selectivity.
FIG. 2 illustrates conversion of HCFC-244bb during dehydrochlorination in ¾" Inconel 625 X 0.035" reactor (450°C, 12 g-organic/h, organic feed: 99.4 GC area% 244bb/0.4% 1233xf GC area%).
FIG. 3 illustrates conversion ofHCFC-244bb and product selectivity during HCFC-244bb dehydrochlorination in reduced ¾" Inconel 625 X 0.035" reactor (480°C, 0 psig, 12 g-organic/h, organic feed: 99.4 GC area% 244bb/0.4% 1233xf GC area%).

### DETAILED DESCRIPTION OF PREFERRED EMBODIMENTS

According to one embodiment, the present invention relates to a manufacturing process for making HFO-1234yf using a starting or intermediate material comprising HCFC-244bb in a reactor that is substantially free of impurities. Applicants have surprisingly found that the presence of impurities, such as metal halides, metal oxides, and carbonaceous materials, decrease the conversion rate and selectivity of HCFC-244bb to HFO-1234yf. While not wishing to be bound by theory, it is believed that certain impurities, such as halides ofNi, Cr, Fe, Mo, Nb, Cu, and Co, are formed through halidation of corresponding metal components of the reactor, and that such impurities catalytically contribute to the formation of HCFO-1233xf. Accordingly, the present invention provides methods of removing impurities from the reactor to improve the overall efficiency of the HFO-1234yf production process.

In certain aspects, the preparation of HFO-1234yf generally includes at least three reaction steps, as follows:
(i) (CX₂=CCl-CH₂X or CX₃-CCl=CH₂ or CX₃-CHCl-CH₂X) + HF → 2-chloro-3,3,3-trifluoropropene (HCFO-1233xf) + HCl in a vapor phase reactor charged with a solid catalyst;
(ii) 2-chloro-3,3,3-trifluoropropene (HCFO-1233xf) + HF → 2-chloro-1,1,1,2-tetrafluoropropane (HCFC-244bb) in a liquid phase reactor charged with a liquid hydrofluorination catalyst; and
(iii)2-chloro-1,1,1,2-tetrafluoropropane (HCFC-244bb) → 2,3,3,3-tetrafluoropropene (HFO-1234yf) in a vapor phase reactor.
wherein X is independently selected from F, Cl, Br, and I, provided that at least one X is not fluorine.

The starting material in the first reaction step is one or more chlorinated compounds according to Formulas I, II, and/or III:

CX₂=CCl-CH₂X (Formula I)

CX₃-CCl=CH₂ (Formula II)

CX₃-CHCl-CH₂X (Formula III)

wherein X is independently selected from F, Cl, Br, and I, provided that at least one X is not fluorine. In certain embodiments, these compounds contain at least one chlorine, a majority of X is chlorine, or all X is chlorine.

In the first step, the starting composition (which, in certain embodiments comprises 1,1,2,3-tetrachloropropene (HCO-1230xa), 2,3,3,3-tetrachloropropene (HCO-1230xf) and/or 1,1,1,2,3-pentachloropropane (HCC-240db)) reacts with anhydrous HF in a first vapor phase reactor (fluorination reactor) to produce a mixture of at least HCFO-1233xf (2-chloro-3,3,3-trifluoropropene) and HCl. The reaction can be carried out at a temperature of about 200-400°C and a pressure of about 0-1.4 MPag (0-200 psig). The effluent stream exiting the vapor phase reactor may optionally comprise additional components, such as un-reacted HF, un-reacted starting compositions, heavy intermediates, HFC-245cb, or the like.

This reaction may be conducted in any reactor suitable for a vapor phase fluorination reaction. The reactor may be constructed from materials which are resistant to the corrosive effects of hydrogen fluoride and catalyst such as Hastalloy, Inconel, Monel. In case of a vapor phase process, the reactor is filled with a vapor phase fluorination catalyst. Any fluorination catalysts known in the art may be used in this process. Suitable catalysts include, but are not limited to chromium, aluminum, cobalt, manganese, nickel and iron oxides, hydroxides, halides, oxyhalides, inorganic salts thereof and their mixtures any of which may be optionally halogenated. Combinations of catalysts suitable for the present invention nonexclusively include Cr₂O₃, FeCl₃/C, Cr₂O₃/Al₂O₃, Cr₂O₃/AlF₃, Cr₂O₃/carbon, CoCl₂/Cr₂O₃/Al₂O₃, NiCl₂/Cr₂O₃/Al₂O₃, CoCl₂/AlF₃, NiCl₂/AlF₃ and mixtures thereof. Chromium oxide/aluminum oxide catalysts are described in U.S. Pat. No. 5,155,082. Chromium (III) oxides such as crystalline chromium oxide or amorphous chromium oxide are preferred with amorphous chromium oxide being most preferred. Chromium oxide (Cr₂O₃) is a commercially available material which may be purchased in a variety of particle sizes. Fluorination catalysts having a purity of at least 98% are preferred. The fluorination catalyst is present in an excess but in at least an amount sufficient to drive the reaction.

This first step of the reaction is not necessarily limited to a vapor phase reaction and may also be performed using a liquid phase reaction or a combination of liquid and vapor phases, such as that disclosed in U.S. Published Patent Application No. 20070197842. It is also contemplated that the reaction can be carried out batch wise, continuous, or a combination of these. For embodiments in which the reaction comprises a liquid phase reaction, the reaction can be catalytic or non-catalytic. Lewis acid catalysts, such as metal-halide catalysts, including antimony halides, tin halides, thallium halides, iron halides, and combinations of two or more of these, may be employed. In certain embodiments, metal chlorides and metal fluorides are employed, including, but not limited to, SbCl₅, SbCl₃, SbF₅, SnCl₄, TiCl₄, FeCl₃ and combinations of two or more of these.

In the second step of the process for forming 2,3,3,3-tetrafluoropropene, HCFO-1233xf is converted to HCFC-244bb. In one embodiment, this step may be performed in the liquid phase in a liquid phase reactor, which may be TFE or PFA-lined. Such a process may be performed in a temperature range of about 70-120°C and about 0.3 - 0.8 Mpag (50-120 psig).

Any liquid phase fluorination catalyst may be used in the invention. A non-exhaustive list include Lewis acids, transition metal halides, transition metal oxides, Group IVb metal halides, a Group Vb metal halides, or combinations thereof. Non-exclusive examples of liquid phase fluorination catalysts are an antimony halide, a tin halide, a tantalum halide, a titanium halide, a niobium halide, and molybdenum halide, an iron halide, a fluorinated chrome halide, a fluorinated chrome oxide or combinations thereof. Specific non-exclusive examples of liquid phase fluorination catalysts are SbCl₅, SbCl₃, SbF₅, SnCl₄, TaCl₅, TiCl₄, NbCl₅, MoCl₆, FeCl₃, a fluorinated species of SbCl₅, a fluorinated species of SbCl₃, a fluorinated species of SnCl₄, a fluorinated species of TaCl₅, a fluorinated species of TiCl₄, a fluorinated species of NbCl₅, a fluorinated species of MoCl₆, a fluorinated species of FeCl₃, or combinations thereof. Antimony pentachloride is most preferred.

These catalysts can be readily regenerated by any means known in the art if they become deactivated. One suitable method of regenerating the catalyst involves flowing a stream of chlorine through the catalyst. For example, from 0.9g to 90g (0.002 to 0.2 lb) per hour of chlorine can be added to the liquid phase reaction for every pound of liquid phase fluorination catalyst. This may be done, for example, for from about 1 to about 2 hours or continuously at a temperature of from about 65°C to about 100°C.

This second step of the reaction is not necessarily limited to a liquid phase reaction and may also be performed using a vapor phase reaction or a combination of liquid and vapor phases, such as that disclosed in U.S. Published Patent Application No. 20070197842. To this end, the HCFO-1233xf containing feed stream is preheated to a temperature of from about 50°C to about 400°C, and is contacted with a catalyst and fluorinating agent. Catalysts may include standard vapor phase agents used for such a reaction and fluorinating agents may include those generally known in the art, such as, but not limited to, hydrogen fluoride.

In the third step of HFO-1234yf production, HCFC-244bb is fed to a second vapor phase reactor (dehydrochlorination reactor) to be dehydrochlorinated to make the desired product 2,3,3,3-tetrafluoropropene (HFO-1234yf). This reactor contains a catalyst that can catalytically dehydrochlorinate HCFC-244bb to make HFO-1234yf.

The catalysts may be metal halides, halogenated metal oxides, neutral (or zero oxidation state) metal or metal alloy, or activated carbon in bulk or supported form. Metal halide or metal oxide catalysts may include, but are not limited to, mono-, bi-, and tri-valent metal halides, oxides and their mixtures/combinations, and more preferably mono-, and bi-valent metal halides and their mixtures/combinations. Component metals include, but are not limited to, Cr³⁺, Fe³⁺, Mg²⁺, Ca²⁺, Ni²⁺, Zn²⁺, Pd²⁺, Li⁺, Na⁺, K⁺, and Cs⁺. Component halogens include, but are not limited to, F⁻, Cl⁻, Br⁻, and I⁻. Examples of useful mono- or bi-valent metal halide include, but are not limited to, LiF, NaF, KF, CsF, MgF₂, CaF₂, LiCl, NaCl, KCl, and CsCl. Halogenation treatments can include any of those known in the prior art, particularly those that employ HF, F₂, HCl, Cl₂, HBr, Br₂, HI, and I₂ as the halogenation source.

Neutral, i.e., zero valent, metals, metal alloys and their mixtures can be used. Useful metals include, but are not limited to, Pd, Pt, Rh, Fe, Co, Ni, Cu, Mo, Cr, Mn, and combinations of the foregoing as alloys or mixtures. The catalyst may be supported or unsupported. Useful examples of metal alloys include, but are not limited to, SS 316, Monel 400, Inconel 825, Inconel 600, and Inconel 625. Such catalysts may be provided as discrete supported or unsupported elements and/or as part of the reactor and/or the reactor walls.

Preferred, but non-limiting, catalysts include activated carbon, stainless steel (e.g., SS 316), austenitic nickel-based alloys (e.g., Inconel 625), nickel, fluorinated 10% CsCl/MgO, and 10% CsCl/MgF₂. A suitable reaction temperature is about 300-550°C and a suitable reaction pressure may be between 0-1 MPag (0-150 psig). The reactor effluent may be fed to a caustic scrubber or to a distillation column to remove the byproduct of HCl to produce an acid-free organic product which, optionally, may undergo further purification using one or any combination of purification techniques that are known in the art.

The reaction may be carried out at a temperature range of from about 200°C to about 800° C, from about 300°C to about 600°C, or from about 400°C to about 500°C. Suitable reactor pressures range from 0-1.4 MPag (0-200 psig), from 0.07-0.7 MPag (10-100 psig), or from 0.14-0.5 MPag (20-70 psig).

In vapor-phase HCFC-244bb dehydrochlorination, HCFC-244bb feed, which can be formed from HCFO-1233xf hydrofluorination as described in US 20090240090, is fed continuously to a vaporizer and the vaporized feed to a reactor. Due to incomplete conversion of HCFO-1233xf and its close boiling point to HCFC-244bb as well as the formation of azeotrope or azeotrope-like composition of HCFC-244bb and HCFO-1233xf under certain conditions, the separation of these two compounds is difficult. For this reason, the HCFC-244bb feed generally contains certain amount of HCFO-1233xf. The dehydrochlorination reaction may be carried out under conditions to attain a HCFC-244bb conversion of about 5% or higher, about 20% or higher, or about 30% or higher. The reaction may be carried out at a temperature in the range of from about 200°C to about 800° C, from about 300°C to about 600°C, or from about 400°C to about 500° C; the reactor pressure may be in the range of from about 0 to about 1 MPag (about 0 psig to about 200 psig), from about 70 to about 700 kPag (about 10 psig to about 100 psig), or from about 140 to about 500 kPag (about 20 to about 70 psig).

In general, the effluent from the dehydrochlorination reactor may be processed to achieve desired degrees of separation and/or other processing. Besides HFO-1234yf produced, the effluent generally contains HCl, unconverted HCFC-244bb, and HCFO-1233xf (which is mainly carried over from the previous step of HCFO-1233xf hydro fluorination). Optionally, HCl is then recovered from the result of the dehydrochlorination reaction. Recovery of HCl is conducted by conventional distillation where it is removed from the distillate. Alternatively, HCl can be recovered or removed by using water or caustic scrubbers. When a water extractor is used HCl is removed as an aqueous solution. When a caustic solution is used, HCl is removed from system as a chloride salt in aqueous solution. After the recovery or removal of HCl, the organic stream may be sent to a distillation column for separation. HFO-1234yf, collected from the overhead of the column, may be sent to another column for further purification, while a fraction of the mixture of HCFO-1233xf and HCFC-244bb, accumulated in the reboiler, may be sent back to the dehydrochlorination reactor for the recycle of HCFC-244bb, and the rest to the HCFO-1233xf hydrofluorination reactor for the recycle of HCFO-1233xf.

Applicants have surprisingly discovered that, during the dehydrochlorination of HCFC-244bb to form HFO-1234yf, the presence of impurities in the reactor decreases the selectivity to HFO-1234yf and increases selectivity toward HCFO-1233xf, which is an undesired byproduct. Applicants have further discovered that the same impurities may also increase catalyst deactivation, thus causing a reduction of the conversion rate. While not intending to be bound by theory, it is believed that metal halides such as NiX₂ (X = F, or Cl), CrX₃, FeX₃, MoX₃, NbX₃, COX₂, and the like, are incidentally formed by halidation of the metal components of the reactor (e.g., Inconel 625), and carbonaceous materials by the pyrolysis of organic compounds present in the reactor. These metal halides, especially trivalent metal halides, act as dehydrofluorination catalysts converting HCFC-244bb to HCFO-1233xf. In other aspects, the metal halides and/or carbonaceous materials block the catalytically active metallic sites converting HCFC-244bb to HFO-1234yf. The present invention provides a solution to this problem by reducing the content of impurities in the reactor, thereby improving HFO-1234yf selectivity and similarly reducing the formation of HCFO-1233xf.

To this end, the impurities in the reactor are removed such that the reactor is substantially free of impurities. As used herein, the term "impurities" includes any compound or combination of compounds that (1) reduce the selectivity of HCFC-244bb to HFO-1234yf, (2) increase selectivity changeover from HFO-1234yf to HCFC-1233xf, and/or (3) decrease the conversion rate of HCC-244bb to HFO-1234yf. Such impurities may include, but are not limited to, metal halides, metal oxides, and carbonaceous materials. As used herein, the definition of "substantially free" means that the amount of impurities is reduced in the reactor so as to measurably improve selectivity of the conversion of HCFC-244bb to HFO-1234yf, decrease selectivity in the conversion of HCFC-244bb to HCFO-1233xf, and/or increase the conversion rate of HCFC-244bb to HFO-1234yf. While the definition of "substantially free" may be as defined herein, in one aspect, the removal of impurities improves selectivity of HCFC-244bb to HFO-1234yf to at least 90% or higher, 95% or higher, or 97% or higher, or 99% or higher, or 99.5% or higher. Selectivity may be calculated by number of moles of product (HFO-1234yf) formed divided by number of moles of reactant consumed or, otherwise, using standard methods known in the art.

The step of removing impurities from the reactor may be accomplished using any method for removing such impurities in accordance with the claims, particularly the impurities provided herein. In one embodiment, it may be achieved by introducing a reducing agent into the reactor under conditions effective to convert any metal halides or metal oxides into metallic metals. Such reducing agents may include, but are not limited to, H₂, NH₃, CO, and combinations of these. This reducing step may be carried out in pure or diluted reducing agent, e.g., hydrogen, at a temperature range of from about 100°C to about 700°C, about 200°C to about 600°C, about 300°C to about 500°C, or about 400°C to about 500°C. Suitable diluents include inert gases such as N₂, Ar, and He. When a diluted reducing agent is used, the dilution can be as high as practically possible, for example, about 0.1% volume of reducing agent. In a preferred embodiment, the concentration of reducing agent after dilution ranges from about 0.5 to about 20 vol%, preferably from about 1 to about 5 vol%, and more preferably from about 2 to about 2.5 vol%.

Another method for removing impurities from the reactor may be achieved by introducing an oxidizing agent into the reactor under conditions effective to burn off the carbonaceous materials in the reactor. The oxidizing agent, for example, can comprise, but is not limited to, H₂O, CO₂, O₂ (oxygen), air, O₃, Cl₂, N₂O, and combinations of these. This oxidation step can be carried out in pure or diluted oxidizing agent, e.g., oxygen at a temperature range of from about 100°C to about 700°C, about 200°C to about 600°C, about 300°C to about 500°C, or about 400°C to about 500°C. When a diluted oxidizing agent is used, the dilution can be as high as practically possible, for example, about 0.1% volume of oxidizing agent. In a preferred embodiment, the concentration of oxidizing agent after dilution ranges from about 0.5 to about 21 vol%, preferably from about 1 to about 5 vol%, and more preferably from about 2 to about 3 vol%. In another preferred embodiment, air is used as oxygen source and diluted air is used. In yet another preferred embodiment, air is used together with steam in a steam-air decoking process, in which air is used to burn off the carbonaceous materials and steam is used to keep the burning temperatures low such that they do not exceed the maximum tolerable temperatures. This step may be used independently or in conjunction with any of the alternative methods herein. In certain embodiments, after the impurities are removed using the oxidizing agent, the reactor is then treated with a reducing agent in accordance with the teachings above.

In yet another embodiment, the step of removing impurities from the reactor may be achieved by physically removing carbonaceous materials and metal halides from the reactor. This physical removal step may include, for example, electrical polishing and mechanical polishing. In another example, these impurity deposits can be washed away with high flow rate of water or steam. Again, this step may be used independently or in conjunction with any of the alternative methods herein.

In certain non-limiting embodiments, the specific steps used in the process of removing the impurities may be directed to improve either selectivity or catalyst activity. With respect to improving selectivity and preventing selectivity changeover, for example, the reducing step may be performed for purposes of substantially removing the impurities from the reactor walls. With respect to catalyst activity, it may be recovered or regenerated by oxidation treatment followed by reduction treatment. Alternatively, either selectivity or catalytic activity may be improved by polishing treatment.

The process of the invention may be employed upon the detection of preset or otherwise determined levels of selectivity, for example, upon reaching an undesired selectivity of HFO-1234_{yf} or HCFO-1233_{xf}. In one embodiment, the invention is directed to a process for improving the selectivity for 2,3,3,3-tetrafluoropropene where the process is as herein described, comprising: (a) providing a reaction to make 2,3,3,3-tetrafluoropropene, the reaction taking place in a reactor, the reaction having a first selectivity for 2,3,3,3-tetrafluoropropene, which first selectivity, in one embodiment, is at least 90% or higher as herein described; (b) detecting a second selectivity for 2,3,3,3-tetrafluoropropene as the reaction proceeds, for example, the second selectivity is lower than the first selectivity, and includes without limitation undesirable selectivity for commercial purposes, which selectivity in one embodiment is lower than 90%, including about 50% or less; (c) ceasing the reaction; (d) removing impurities from a reactor such that the reactor is substantially free of impurities; removal of impurities in this regard includes removal by (i) introducing a reducing agent, (ii) introducing an oxidizing agent, or (iii) by mechanical removal, or by combinations of (i), (ii) or (iii) as herein described. In one embodiment, removal of impurities is by introducing a reducing agent into the reactor under conditions effective to reduce the impurities; and (e) resuming the reaction after the removal of impurities step. In one embodiment in this regard, the resumed reaction has a third selectivity for 2,3,3,3-tetrafluoropropene that is detected, the third selectivity for 2,3,3,3-tetrafluoropropene being greater than the second selectivity for 2,3,3,3-tetrafluoropropene; in one practice in this regard, the third selectivity for 2,3,3,3-tetrafluoropropene is substantially the same as the first selectivity . In one practice of this embodiment, the process further comprises, after step (c) and prior to introducing the reducing agent, introducing an oxidizing agent into the reactor under conditions effective to oxidize the impurities. In another practice of this embodiment, the process further comprising purging the reactor with an inert gas after the impurities are oxidized and prior to introducing the reducing agent. In another practice of this embodiment, physical removal of impurities, e.g. polishing and hydraulic method and the like, can take place prior to or after reduction and/or prior to or after purging and/or prior to or after oxidation.

The following are examples of the invention and are not to be construed as limiting.

### EXAMPLES

### Example 1

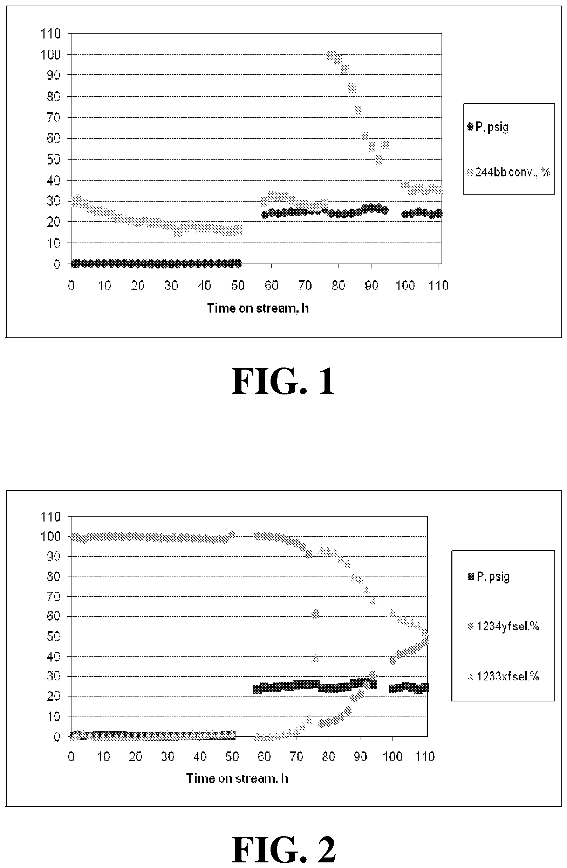

This example illustrates the occurrence of selectivity changeover from HFO-1234yf to HCFO-1233xf during vapor phase dehydrchlorination reaction of HCFC-244bb. A cylindrical Inconel 625 reactor of ¾" diameter immersed into a 3-zone electrical furnace was used. Process temperatures were recorded using a multi-point thermocouple placed inside the reactor. The inner wall of Inconel 625 reactor serves as dehydrochlorination catalyst. A feed containing 99.4 GC area% HCFC-244bb and 0.4 GC area% HCFO-1233xf was fed into the bottom of the vertically mounted reactor and was vaporized before reaching reaction zone. The reaction was conducted under conditions of 450°C, 0 and 170 kPag (0 and 25 psig), and 12 g-organic/h. Effluent gases were passed through a gas sampling tube so that the progress of the reaction was monitored periodically via GC analysis of the contents of the gas sampling tube. As shown in FIG. 1 and FIG. 2, after about 20 hours on stream at 450°C and about 170 kPag (about 25 psig), HCFC-244bb conversion was dramatically increased from about 30% initially to almost 100% while HFO-1234yf selectivity was dramatically decreased from about 99.5% initially to below 10% (simultaneously, HCFO-1233xf selectivity was increased from below 0.5% to above 90%), indicating the occurrence of selectivity changeover from HFO-1234yf to HCFO-1233xf. After that, even though the selectivity to HCFO-1233xf started to drop, it remained at about 50% at the end of the experiment.

### Example 2

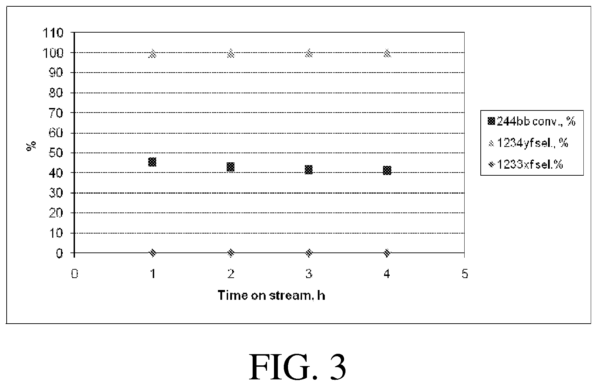

This example is a continuation of Example 1. After the occurrence of selectivity changeover from HFO-1234yf to HCFO-1233xf, the reactor was reduced in hydrogen flow (200 ml/min) for 2 hours at 480°C and then the reaction of HCFC-244bb dehydrochlorination was re-started. As shown in Figure 3, the selectivity to HFO-1234yf was almost 100% and the conversion of HCFC-244 was above 40% after reduction treatment.

## Claims

1. A process for preparing 2,3,3,3-tetrafluoropropene comprising:
(a) providing a reaction to make 2,3,3,3-tetrafluoropropene by dehydrochlorination of 2-chloro-1,1,1 ,2-tetrafluoropropane, the reaction taking place in a reactor, the reaction having a first selectivity for 2,3,3,3-tetrafluoropropene;
(b) detecting a second selectivity for 2,3,3,3-tetrafluoropropene as the reaction proceeds;
(c) ceasing the reaction;
(d) removing impurities from said reactor by introducing a reducing agent or an oxidizing agent or by mechanical means under conditions effective to reduce the impurities and improve selectivity to 2,3,3,3-tetrafluoropropene; and
(e) resuming the reaction.

2. The process of claim 1, wherein the impurities in the reactor are selected from the group consisting of metal halides, metal oxides, and carbonaceous materials, optionally wherein the metal halides comprise halides of Ni, Cr, Fe, Mo, Nb, Cu, and Co.

3. The process of claims 1-2, wherein the first selectivity for 2,3,3,3-tetrafluoropropene is at least 90% or higher.

4. The process of claims 1-3, wherein the second selectivity for 2,3,3,3-tetrafluoropropene is lower than 90%, or 50% or lower.

5. The process of claims 1-4, wherein the resumed reaction has a third selectivity for 2,3,3,3-tetrafluoropropene that is detected, the third selectivity being greater than the second selectivity.

6. The process of any one of claims 1-2, wherein the step of removing impurities from the reactor comprises introducing a reducing agent into the reactor under conditions effective to convert any metal halides or metal oxides into metallic metals, preferably wherein the reducing agent is selected from the group consisting of H₂, NH₃, CO, C₁-C₁₂ hydrocarbons, and combinations of these.

7. The process of any one of claims 1-2, wherein the step of removing impurities from the reactor comprises introducing an oxidizing agent into the reactor under conditions effective to burn off the carbonaceous materials in the reactor, preferably wherein the oxidizing agent is selected from the group consisting of H₂O, CO₂, O₂, air, O₃, Cl₂, N₂O, and combinations of these, and more preferably wherein the oxidizing agent comprises oxygen.

8. The process of any one of claims 1-2, wherein the step of removing impurities from the reactor comprises physically removing carbonaceous materials, metal oxides, and metal halides from the reactor, preferably wherein the step of physically removing the carbonaceous materials, metal oxides, and metal halides from the reactor is selected from the group consisting of electrical polishing, mechanical polishing, hydraulic methods, and combinations of these.

9. The process of any one of claims 1-2 or 6-8, wherein the selectivity to 2,3,3,3-tetrafluoropropene is at least 90% or higher, preferably at least 95% or higher, and more preferably at least 97% or higher.

10. A process for preparing 2,3,3,3-tetrafluoropropene comprising:
(a) providing a reaction to make 2,3,3,3-tetrafluoropropene, the reaction taking place in a reactor, the reaction having a first selectivity for 2,3,3,3- tetrafluoropropene;
(b) detecting a second selectivity for 2,3,3,3-tetrafluoropropene as the reaction proceeds;
(c) ceasing the reaction;
(d) removing impurities from said reactor such that the reactor is substantially free of impurities by introducing a reducing agent into the reactor to measurably improve the 2,3,3,3-tetrafluoropropene production rate and/or the selectivity of the conversion of 2-chloro-1,1,1,2-tetrafluoropropane to 2,3,3,3-tetrafluoropropene, or to decrease the conversion of 2-chloro-1,1,1,2-tetrafluoropropane to 2-chloro-3,3,3-trifluoropropene; and
(e) resuming the reaction.

11. The process of claim 10 further comprising, after step (c) and prior to introducing the reducing agent, introducing an oxidizing agent into the reactor under conditions effective to oxidize the impurities.

12. The process of claim 11, further comprising purging the reactor with an inert gas after the impurities are oxidized and prior to introducing the reducing agent.

13. The process of claim 10, wherein the resumed reaction has a third selectivity for 2,3,3,3-tetrafluoropropene, the third selectivity for 2,3,3,3-tetrafluoropropene being greater than the second selectivity for 2,3,3,3-tetrafluoropropene, preferably wherein the third selectivity to 2,3,3,3-tetrafluoropropene is at least 90% or higher, more preferably at least 95% or higher, and even more preferably at least 97% or higher.

14. The process of claim 11, wherein the oxidizing agent is selected from the group consisting of H₂O, CO₂, O₂, air, O₃, Cl₂, N₂O and combinations of these.

15. The process of claim 10, wherein the reducing agent is selected from the group consisting of H₂, NH₃, CO, C₁-C₁₂ hydrocarbons, and combinations of these.

## Patentansprüche

1. Verfahren zur Herstellung von 2,3,3,3-Tetrafluorpropen, umfassend:
(a) Herbeiführen einer Reaktion zur Herstellung von 2,3,3,3-Tetrafluorpropen durch Dehydrochlorierung von 2-Chlor-1,1,1,2-Tetrafluorpropan, wobei die Reaktion in einem Reaktor stattfindet und wobei die Reaktion eine erste Selektivität für 2,3,3,3-Tetrafluorpropen hat,
(b) Erkennen einer zweiten Selektivität für 2,3,3,3-Tetrafluorpropen mit fortschreitender Reaktion,
(c) Beenden der Reaktion,
(d) Entfernen von Verunreinigungen aus dem Reaktor durch Zufügen eines Reduktionsmittels oder eines Oxidationsmittels oder durch mechanische Mittel unter Bedingungen, die wirksam sind um die Verunreinigungen zu vermindern und die Selektivität für 2,3,3,3-Tetrafluorpropen zu verbessern, und
(e) Wiederaufnahme der Reaktion.

2. Verfahren nach Anspruch 1, wobei die Verunreinigungen im Reaktor ausgewählt sind aus der aus Metallhalogeniden, Metalloxiden und kohlenstoffhaltigen Materialien bestehenden Gruppe, wobei wahlweise die Metallhalogenide Halogenide von Ni, Cr, Fe, Mo, Nb, Cu und Co umfassen.

3. Verfahren nach den Ansprüchen 1 bis 2, wobei die erste Selektivität für 2,3,3,3-Tetrafluorpropen mindestens 90 % oder höher ist.

4. Verfahren nach den Ansprüchen 1 bis 3, wobei die zweite Selektivität für 2,3,3,3-Tetrafluorpropen weniger als 90 % oder 50 % oder weniger ist.

5. Verfahren nach den Ansprüchen 1 bis 4, wobei die wiederaufgenommene Reaktion eine dritte Selektivität für 2,3,3,3-Tetrafluorpropen hat, die erkannt wird, wobei die dritte Selektivität größer als die zweite Selektivität ist.

6. Verfahren nach einem der Ansprüche 1 bis 2, wobei der Schritt Entfernen der Verunreinigungen aus dem Reaktor die Einführung eines Reduktionsmittels in den Reaktor unter Bedingungen umfasst, welche zur Umwandlung eines beliebigen der Metallhalogenide oder Metalloxide in metallische Metalle wirksam sind, wobei bevorzugt das Reduktionsmittel aus der aus H₂, NH₃, CO, C₁- bis C₁₂-Kohlenwasserstoffe und Kombinationen aus diesen bestehenden Gruppe ausgewählt ist.

7. Verfahren nach einem der Ansprüche 1 bis 2, wobei der Schritt Entfernen der Verunreinigungen aus dem Reaktor die Einführung eines Oxidationsmittels in den Reaktor unter Bedingungen umfasst, die wirksam sind, um das kohlenstoffhaltige Material im Reaktor zu verbrennen, wobei bevorzugt das Oxidationsmittel aus der aus H₂O, CO₂, O₂, Luft, O₃, Cl₂, N₂O und Kombinationen aus diesen bestehenden Gruppe ausgewählt ist, wobei mehr bevorzugt das Oxidationsmittel Sauerstoff umfasst.

8. Verfahren nach einem der Ansprüche 1 bis 2, wobei der Schritt Entfernen der Verunreinigungen aus dem Reaktor das physikalische Entfernen der kohlenstoffhaltigen Materialien, Metalloxide und Metallhydride aus dem Reaktor umfasst, wobei bevorzugt der Schritt physikalisches Entfernen der kohlenstoffhaltigen Materialien, Metalloxide und Metallhalogenide aus dem Reaktor aus der aus Elektropolieren, mechanischem Polieren, hydraulischen Verfahren und Kombinationen aus diesen bestehenden Gruppe ausgewählt ist.

9. Verfahren nach einem der Ansprüche 1 bis 2 oder 6 bis 8, wobei die Selektivität für 2,3,3,3-Tetrafluorpropen mindestens 90 % oder höher, bevorzugt mindestens 95 % oder höher, mehr bevorzugt mindestens 97 % oder höher ist.

10. Verfahren zur Herstellung von 2,3,3,3-Tetrafluorpropen, umfassend:
(a) Herbeiführen einer Reaktion zur Herstellung von 2,3,3,3-Tetrafluorpropen, wobei die Reaktion in einem Reaktor stattfindet und wobei die Reaktion eine erste Selektivität für 2,3,3,3-Tetrafluorpropen hat,
(b) Erkennen einer zweiten Selektivität für 2,3,3,3-Tetrafluorpropen mit fortschreitender Reaktion,
(c) Beenden der Reaktion,
(d) Entfernen von Verunreinigungen aus dem Reaktor, sodass der Reaktor im Wesentlichen frei von Verunreinigungen ist, durch Einführen eines Reduktionsmittels in den Reaktor zur messbaren Verbesserung der Produktionsgeschwindigkeit von 2,3,3,3-Tetrafluorpropen und/oder der Selektivität der Umwandlung von 2-Chlor-1,1,1,2-Tetrafluorpropan in 2,3,3,3-Tetrafluorpropen, oder um die Umwandlung von 2-Chlor-1,1,1,2-Tetrafluorpropan in 2-Chlor-3,3,3-Tetrafluorpropen zu verringern, und
(e) Wiederaufnahme der Reaktion.

11. Verfahren nach Anspruch 10, ferner nach dem Schritt (c) und vor dem Einführen des Reduktionsmittels den Schritt Einführen eines Oxidationsmittels in den Reaktor unter Bedingungen, die zum Oxidieren der Verunreinigungen wirksam sind, umfassend.

12. Verfahren nach Anspruch 11, ferner das Spülen des Reaktors mit einem Inertgas nach dem Oxidieren der Verunreinigungen und vor dem Einführen des Reduktionsmittels umfassend.

13. Verfahren nach Anspruch 10, wobei die wieder aufgenommene Reaktion eine dritte Selektivität für 2,3,3,3-Tetrafluorpropen aufweist, wobei die dritte Selektivität für 2,3,3,3-Tetrafluorpropen größer als die zweite Selektivität für 2,3,3,3-Tetrafluorpropen ist, wobei bevorzugt die dritte Selektivität für 2,3,3,3-Tetrafluorpropen mindestens 90 % oder höher, mehr bevorzugt mindestens 95 % oder höher und noch mehr bevorzugt wenigstens 97 % oder höher ist.

14. Verfahren nach Anspruch 11, wobei das Oxidationsmittel aus der aus H₂O, CO₂, O₂, Luft, O₃, Cl₂, N₂O und Kombinationen aus diesen bestehenden Gruppe ausgewählt ist.

15. Verfahren nach Anspruch 10, wobei das Reduktionsmittel aus der aus H₂, NH₃, CO, C₁-bis C₁₂-Kohlenwasserstoffen und Kombination aus diesen bestehenden Gruppe ausgewählt ist.

## Revendications

1. Procédé de préparation de 2,3,3,3-tétrafluoropropène comprenant des étapes consistant à :
(a) préparer du 2,3,3,3-tétrafluoropropène par déshydrochloration de 2-chloro-1,1,1,2-tétrafluoropropane, cette réaction étant effectuée dans un réacteur, et ayant une première sélectivité pour le 2,3,3,3-tétrafluoropropène,
(b) détecter une seconde sélectivité pour le 2,3,3,3-tétrafluoropropène lors de la progression de la réaction,
(c) arrêter la réaction,
(d) éliminer des impuretés du réacteur en introduisant un agent réducteur ou un agent oxydant ou par des moyens mécaniques dans des conditions efficaces pour réduire les impuretés et améliorer la sélectivité pour le 2,3,3,3-tétrafluoropropène, et
(e) recommencer la réaction.

2. Procédé conforme à la revendication 1,
selon lequel les impuretés présentes dans le réacteur sont des impuretés du groupe formé par des halogénures métalliques, des oxydes métalliques, des matériaux carbonés et le cas échéant, les halogénures métalliques sont des halogénures de Ni, Cr, Fe, Mo, Nb, Cu et Co.

3. Procédé conforme à la revendication 1 ou 2,
selon lequel la première sélectivité pour le 2,3,3,3-tétrafluoropropène est d'au moins 90% ou supérieure.

4. Procédé conforme aux revendications 1 à 3,
selon lequel la seconde sélectivité pour le 2,3,3,3-tétrafluoropropène est inférieure à 90% ou à 50% ou inférieure.

5. Procédé conforme aux revendications 1 à 4,
selon lequel la réaction recommencée a une troisième sélectivité pour le 2,3,3,3-tétrafluoropropène qui est détectée, cette troisième sélectivité étant supérieure à la seconde sélectivité.

6. Procédé conforme à l'une quelconque des revendications 1 et 2,
selon lequel l'étape d'élimination des impuretés du réacteur comporte une étape d'introduction d'un agent réducteur dans le réacteur sous des conditions efficaces pour transformer des halogénures métalliques ou des oxydes métalliques quelconques en des métaux métalliques, et de préférence, l'agent réducteur est choisi dans le groupe formé par H₂, NH₃, CO, des hydrocarbures en C₁-C₁₂ et leurs combinaisons.

7. Procédé conforme à l'une quelconque des revendications 1 et 2,
selon lequel l'étape d'élimination des impuretés du réacteur comprend une étape d'introduction d'un agent oxydant dans le réacteur dans des conditions efficaces pour brûler les matériaux carbonés présents dans le réacteur et de préférence, l'agent oxydant est choisi dans le groupe formé par H₂O, CO₂, O₂, l'air, O₃, Cl₂, N₂O et leurs combinaisons, et de façon plus préférentielle, l'agent oxydant renferme de l'oxygène.

8. Procédé conforme à l'une quelconque des revendications 1 et 2,
selon lequel l'étape d'élimination des impuretés du réacteur comprend une étape d'élimination physique de matériaux carbonés d'oxydes métalliques et d'halogénures métalliques du réacteur et de préférence l'étape d'élimination physique de matériaux carbonés, d'oxydes métalliques et d'halogénures métalliques du réacteur est sélectionnée dans le groupe formé par un polissage électrique, un polissage hydraulique, un processus hydraulique et leurs combinaisons.

9. Procédé conforme à l'une quelconque des revendications 1 et 2, ou 6 à 8,
selon lequel la sélectivité pour le 2,3,3,3-tétrafluoropropène est d'au moins 90% ou supérieure de préférence d'au moins 95% ou supérieure et de façon plus préférentielle, d'au moins 97% ou supérieure.

10. Procédé de préparation de 2,3,3,3-tétrafluoropropène comprenant des étapes consistant à :
(a) mettre en œuvre une réaction pour obtenir du 2,3,3,3-tétrafluoropropène, cette réaction étant effectuée dans un réacteur, et ayant une première sélectivité pour le 2,3,3,3-tétrafluoropropène,
(b) détecter une seconde sélectivité pour le 2,3,3,3-tétrafluoropropène lors de la progression de la réaction,
(c) arrêter la réaction,
(d) éliminer des impuretés du réacteur de sorte que le réacteur soit essentiellement exempt d'impuretés en utilisant un agent réducteur dans le réacteur pour augmenter de façon mesurable le taux de production de 2,3,3,3-tétrafluoropropène et/ou la sélectivité de la conversion du 2-chloro-1,1,1,2-tetrafluoropropane en 2,3,3,3-tetrafluoropropène ou pour diminuer la conversion du 2-chloro-1,1,1,2-tetrafluoropropane en 2-chloro-3,3,3-trifluoropropène, et
(e) recommencer la réaction.

11. Procédé conforme à la revendication 10,
comprenant en outre, après l'étape (c) et avant l'introduction de l'agent réducteur, une étape d'introduction d'un agent oxydant dans le réacteur, dans des conditions efficaces pour oxyder les impuretés.

12. Procédé conforme à la revendication 11,
comprenant en outre une étape consistant à purger le réacteur avec un gaz inerte après que les impuretés aient été oxydées et avant l'introduction de l'agent réducteur.

13. Procédé conforme à la revendication 10,
selon lequel la réaction recommencée a une troisième sélectivité pour le 2,3,3,3-tétrafluoropropène, cette troisième sélectivité pour le 2,3,3,3-tétrafluoropropène étant supérieure à la seconde sélectivité pour le 2,3,3,3-tétrafluoropropène, de préférence, la troisième sélectivité pour le 2,3,3,3-tétrafluoropropène étant d'au moins 90% ou supérieure, de façon plus préférentielle, d'au moins 95% ou supérieure et de façon encore plus préférentielle, d'au moins 97% ou supérieure.

14. Procédé conforme à la revendication 11,
selon lequel l'agent oxydant est choisi dans le groupe formé par H₂O, CO₂, O₂, l'air, O₃, Cl₂, N₂O et leurs combinaisons.

15. Procédé conforme à la revendication 10,
selon lequel l'agent réducteur est choisi dans le groupe formé par H₂, NH₃, CO, des hydrocarbures en C₁-C₁₂ et leurs combinaisons.
